# EUROPEAN PATENT SPECIFICATION

(11) **EP 2 217 314 B1**
(45) Date of publication and mention of the grant of the patent: **17.07.2013**
(21) Application number: 08855183.3
(22) Date of filing: 26.11.2008
(51) Int. Cl.: A61M 25/00

(54) **DRAINAGE CATHETER**
DRAINAGEKATHETER
CATHÉTER DRAINANT

(30) Priority: 29.11.2007 US 991097 P
(43) Date of publication of application: 18.08.2010
(60) Divisional application: 13168109.0
(73) Proprietor: Boston Scientific Scimed, Inc., Maple Grove, MN 55311-1566 (US)
(72) Inventor: CHEVALIER, Jr., Raymond, Elletsville Indiana 47429 (US); MILLER, Daniel, St. Louis Missouri 63104 (US); PICKETT, Steve, Spencer Indiana 47460 (US); WIESE, Carla, Cincinnati Ohio 45236 (US); SHERWOOD, Leslie, P., Elletsville Indiana 47429 (US)
(74) Representative: Pfenning, Meinig & Partner GbR
(86) International application number: PCT/US2008/084875
(87) International publication number: WO 2009/070682

(56) References cited:
- WO-A-2008/091652
- US-A- 5 205 830
- US-A- 5 472 435
- US-A- 5 489 269
- US-A1- 2002 091 352
- US-A1- 2003 153 873
- US-A1- 2006 258 982

## Description

### Background Information

Drainage catheters are often inserted percutaneously into abscesses or other structures to drain fluids. The shafts of these catheters are formed in a variety of sizes, durometers, and coatings. Holes located at the distal end of a typical drainage catheter allow fluids surrounding the catheter to enter a lumen through which they flow to the proximal end of the catheter out of the body. To enhance patient comfort, these catheters are generally made of soft biocompatible plastic. The soft material generally results in low column strength, so that a stiffening member is often inserted into a drainage catheter to allow it to be pushed into the body to a desired location. The stiffening member often abut s a surface near the distal end of the lumen at which the lumen tapers or steps down from a larger diameter, proximal portion to a reduced diameter, distal portion.

US 2002/091352 A1 discloses a catheter for delivering and withdrawing fluid from a patient. A stiffening insert is positioned in the distal tip portion of the catheter body and has a stiffness greater than the stiffness of the distal tip portion to facilitate entry into the abdominal cavity of the patient.

US 2003/0153873 A1 discloses a hard tip over-the-needle intravenous catheter comprising a discrete segment of hard tubing that is press fit into the interior of the distal end of the soft catheter.

US 2006/0258982 A1 refers to a catheter tip assembly comprising an outer tip portion and an inner tip portion consisting of different materials.

US-A-5 489 269 shows a hard tip drainage catheter for direct and percentaneous insertion into the cavity of a patient with the aid of a stiffening cannula and a trocer introducer stylet positioned in the passage of the drainage catheter.

US 5 472 435 A relates to a kink resistant drainage catheter comprising a tapered tip which is provided with a cannula stop which prevents a cannula, which is inserted at the proximal end of a LUER connector, from exiting the distal end of the catheter.

US-A-5 205 830 describes a catheter with a removable assembly comprising a stiffening cannula, hollow dilator and wire guide for facilitating insertion. The distal tip of the stiffening cannula interfaces with a shoulder within the catheter, and insertion is accomplished by application of axial pressure on the cannula.

### Summary of the Invention

The present invention is directed to a catheter including a shaft formed of a first material, the shaft including a shaft lumen extending therethrough from a proximal opening to a distal end of the shaft and an abutting member coupled to the distal end of the shaft, the abutting member being formed of a second material stiffer than the first material and forming an abutting surface at least partially covering a distal end of the shaft lumen in combination with a distal tip coupled to the abutting member, the distal tip including a tip lumen extending therethrough to a distal opening, wherein the abutting member extends through the distal tip to end at the distal opening of the distal tip.

### Brief Description of the Drawings

Figure 1 shows a drainage catheter assembly according to the invention;
Figure 2 shows a cross-sectional view of a catheter according to a first embodiment of the present invention; and
Figure 3 shows a cross-sectional view of a catheter according to a second embodiment of the present invention.

### Detailed Description

The present invention may be further understood with reference to the following description and the appended drawings, wherein like elements are referred to with the same reference numerals. The invention relates to devices for draining fluid from the body. More specifically, the invention relates to a novel construction for a drainage catheter.

Drainage catheters typically are made of either a single material, or with a tip section separate from the catheter shaft. The separate tip section may be made of either a material with a different durometer than the catheter shaft, or a different material altogether.

As described above, stiffening members are often inserted into drainage catheters to aid in insertion. However, as the distal end of such a stiffening member is pressed into the catheter lumen, frictional drag along the outside of the catheter body may result in stretching of the catheter and/or the deformation of the material at the end of the lumen contacted by the stiffening member. In some cases, the stiffening member may become lodged within the tip section, preventing the removal of the stiffening member from the catheter. In such cases, the entire catheter must be removed and replaced, increasing discomfort and the time required for the procedure.

The catheter according to the present invention includes an abutting structure within the distal end of the lumen formed of a material having a higher durometer than that of the material of which the rest of the catheter is formed. The increased durometer of the abutting structure reduces the likelihood of the stiffening member becoming stuck within the tip section. The abutting structure is preferably formed of material of a higher modulus of elasticity than the material of which a shaft of the catheter is formed and may, more preferably be a dissolvable material as described in U.S. Patents 5,401,257 and 5,049,138.

The abutting structure according to the embodiments of this invention may be fused to the catheter shaft material within the tip section as part of the tipping process by, for example, mechanical bonding (e.g., press fit, or snap fit) and/or chemical bonding (e.g., heat fusion, adhesives, or solvents). Alternatively, the abutting structure may be added to the catheter shaft material in a process other than the tipping process. These alternative processes may include a separate adhesive process, a solvent bonding process, a mechanical fit process, or an over-molding process. An outer diameter of the abutting structure preferably mates with an inner diameter of a portion of the catheter shaft within which it is to reside while an inner diameter of the abutting structure is preferably substantially a desired inner diameter of a portion of the lumen extending through the tip section so that the abutting structure does not interfere with fluid flow through the lumen.

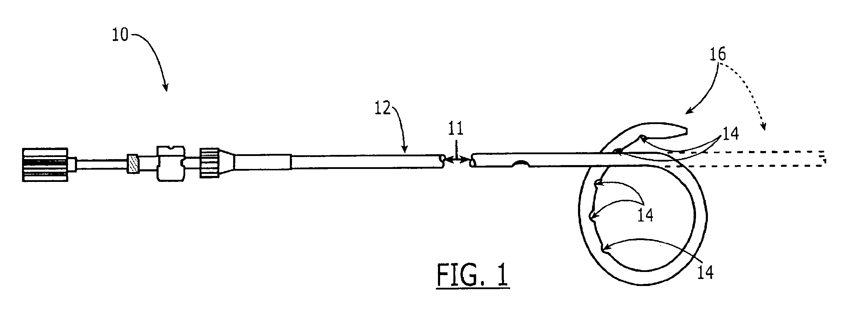

As shown in Fig. 1, a drainage catheter assembly 10 according to the present invention includes a lumen 11 extending through a shaft 12 from the distal end to the proximal end thereof. Generally, multiple holes 14 near the distal end of the catheter shaft 12 allow fluids to drain out of the body. As would be understood by those skilled in the art, in order to assist in maintaining the catheter at a desired location (e.g., within a chamber of a lumen) the tip section 16 of the catheter shaft 12 may have a curled, 'pigtail' shape which anchors the drainage catheter assembly 10 at the outlet from the organ chamber to a lumen through which the catheter assembly 10 was introduced thereinto.

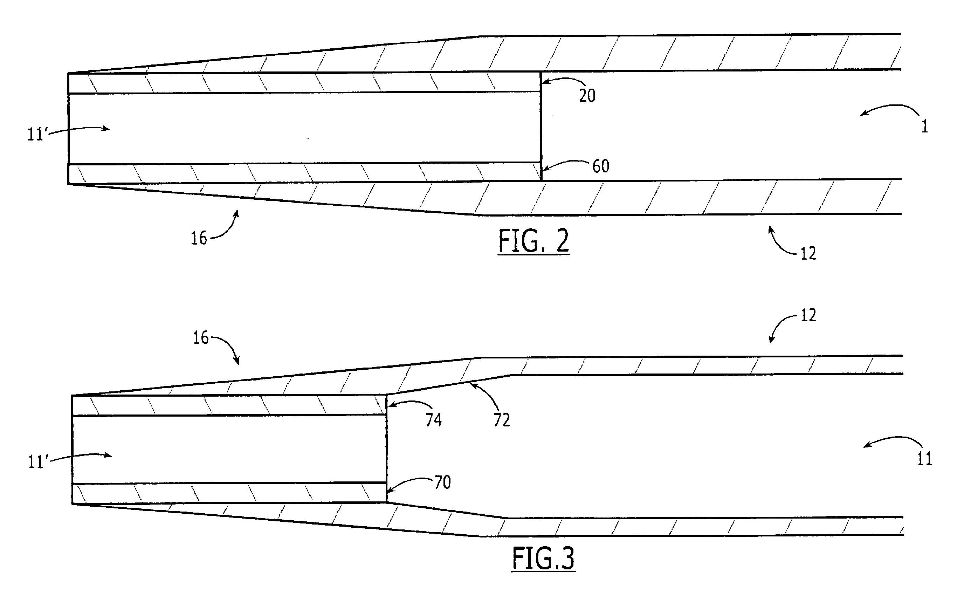

As shown in Fig. 2, a tip 16 including an abutting structure 60 according to a first embodiment of the invention forms a stepped transition 20 in the lumen 11. The abutting structure 60 is formed as a ring mounted within the lumen 11 with the abutting structure 60 being formed of a material stiffer than the material of which the rest of the catheter 10 is formed. An outer diameter of the abutting structure 60 is substantially equal to an inner diameter of the lumen 11 to mate with the inner surface of the catheter shaft 12. The abutting structure 60 extends through the entire tip section 16 to end at the distal opening of the catheter 10. The inner diameter of the abutting structure 60 defines a narrower inner diameter lumen portion 11' of the lumen 11 extending through the tip section 16. Furthermore, a proximal end of the abutting structure 60 defines an increased stiffness stepped transition 20' within the lumen portion 11'. Thus, when a stiffening member is pushed against the stepped transition 20, a material of the stepped transition 20 is less likely to deform, thus reducing the likelihood of the stiffening member becoming lodged within the tip section 16, as those skilled in the art will understand. The catheter 10 and,the abutting structure 60 may be formed of one of the same material and different materials. The materials for either one may include, for example, any type of thermoplastic polyurethane or low density polyurethane, silicone, ethylene vinyl acetate (EVA), or polyvinyl chloride (PVC), etc. The materials may also include a filler such as, for example, a radiopacifier or anti-microbial agent. The abutting structure may each have a different hardness depending on the material chosen. If a material of the abutting structure 60 is the same as that of the catheter 10, it may only need to be of higher durometer than that of the shaft 12 in order to function as indicated above. Alternatively, the abutting surface may be formed of high density polyethylene (HDPE), nylon, polycarbonate, acrylonitrile butadiene styrene (ABS) and aluminum. Catheters made of thermoplastic polyurethane are commonly found in the durometer range of Shore 72A - Shore 84D, as would be understood by those skilled in the art.

As shown in Fig. 3, a tip 16 including an abutting structure 70 according to a second embodiment of the invention is mounted distally of a tapered tip section 72 of the lumen 11. The abutting structure 70 is formed as a ring of a material stiffer than that of which the rest of the catheter 10 is formed and is mated within the reduced diameter lumen portion 11'. The tapered tip section 72 reduces the diameter of lumen 11 in a distal direction and the abutting structure 70 forms a further stepped reduction in this diameter. The abutting structure 70 according to this embodiment extends through the tip 16 from the distal end of the tapered tip section 72 to the distal end of the catheter 10. As in the previous embodiments, the cuter diameter of the abutting structure 70 is substantially equal to the inner diameter of the lumen portion 11' (i.e., less than this inner diameter of the distal portion of the lumen 11 by only a tolerance amount necessary to facilitate insertion therein). The portion of the lumen 11 extending through the abutting structure 70 may have a substantially constant diameter along its length. Alternatively, the lumen portion 11' extending through the abutting structure 70 may comprise a tapered diameter. In another alternate embodiment, the tapered tip section 72 may extend along all or a part of the length of the abutting structure 70 with a corresponding taper on an outer surface of the abutting structure 70. As described above, the abutting structure 70 increases the stiffness of the tapered tip section 72 to reduce the likelihood that of a stiffening member being lodged within the tip section 16 or plastically.deforming the tip section 16.

The abutting structures of the catheters according to the embodiments of the present invention are shown in the appended figures as rings which substantially match the internal shape of the lumen of the catheter. However, alternative designs other than rings (e.g., projections into the lumen) may also be used so long as they provide a stiffer surface for engaging a stiffening member inserted into the catheter. These shapes preferably conform to the shape of the lumen of a single lumen catheter. However, those skilled in the art will understand that for multiple lumen catheters, the shape of an abutting surface may conform to a shape of an outer wall of the catheter, to the shapes of the lumens themselves or may simply form projections into the lumens against which the stiffening member(s) will push. Additionally, the abutting structures may be a variety of sizes (e.g., widths, thicknesses, or diameters) for use in a variety of catheters, for extra stiffening capacity, or for ease of manufacture. Further, one or more abutting structures may be added to multi-lumen drainage catheters as well.

The concept of adding a stiffer material to a catheter shaft may also be used for other products (e.g., venous access catheters such as dialysis, tunneled central, and PICC (peripherally inserted central catheters)) that have flexible shafts into which an insertion tool or other device is inserted to impart a force to the product through contact with an internal structure which might be damaged if not formed of a material more stiff than that of which the rest of the catheter is formed. For example, such a construction may be employed for pacemaker leads, aneurism coils, removable core guidewires, introducer sheaths for vascular access, etc. Further, the secondary material may be added along the entire length of the shaft or only along any portion of the shaft as desired, such as the tip section as shown in the exemplary embodiments.

The present invention has been described with reference to specific embodiments, and more specifically to an abutting structure for use with drainage catheters. However, other embodiments may be devised that are applicable to other medical devices and procedures, without departing from the scope of the invention. Accordingly, various modifications and changes may be made to the embodiments, without departing from the broadest scope of the present invention as set forth in the claims that follow. The specification and drawings are accordingly to be regarded in an illustrative rather than restrictive sense.

## Claims

1. A catheter (10) including:
a shaft (12) formed of a first material, the shaft (12) including a shaft lumen (11) extending therethrough from a proximal opening to a distal end of the shaft (12);
an abutting member (60, 70) coupled to the distal end of the shaft (12), the abutting member (60, 70) being formed of a second material stiffer than the first material and forming an abutting surface at least partially covering a distal end of the shaft lumen (11); and
a distal tip (16) coupled to the abutting member (60, 70), the distal tip (16) including a tip lumen (11') extending therethrough to a distal opening,
**characterized in that**
the abutting member (60, 70) extends through the distal tip (16) to end at the distal opening of the distal tip (16).

2. The catheter of claim, 1, wherein an inner diameter of the abutting member lumen (11') is smaller than an inner diameter of the shaft lumen (11).

3. The catheter of claim 2, wherein the distal tip (16) is formed of the first material.

4. The catheter of claim 1, wherein the distal tip (16) is formed of a third material, the first material being stiffer than the third material.

5. The catheter of claim 1, wherein the shaft lumen (11) includes a tapered transition (72) immediately proximal to the abutting member (70), the inner diameter of a portion of the shaft lumen (11) remaining substantially constant from the proximal opening to a proximal end of the tapered transition (72) and gradually decreasing through the tapered transition (72).

6. The catheter of claim 5, wherein an inner diameter of the abutting member lumen (11') is substantially constant along its length.

## Patentansprüche

1. Katheter (10), enthaltend:
eine Welle (12), die aus einem ersten Material gebildet ist, welche Welle (12) ein Wellenlumen (11) enthält, das sich von einer proximalen Öffnung zu einem distalen Ende der Welle (12) durch diese erstreckt;
ein Anlageteil (60, 70), das mit dem distalen Ende der Welle (12) gekoppelt ist, wobei das Anlageteil (60, 70) aus einem zweiten Material gebildet ist, das steifer als das erste Material ist, und eine Anlagefläche bildet, die zumindest teilweise ein distales Ende des Wellenlumens (11) überdeckt; und
eine distale Spitze (16), die mit dem Anlageteil (60, 70) gekoppelt ist, wobei die distale Spitze (16) ein Spitzenlumen (11') enthält, das sich durch diese bis zu einer distalen Öffnung erstreckt,
**dadurch gekennzeichnet, dass**
das Anlageteil (60, 70) sich durch die distale Spitze (16) erstreckt, um an der distalen Öffnung der distalen Spitze (16) zu enden.

2. Katheter nach Anspruch 1, bei dem ein innerer Durchmesser des Lumens (11') des Anlageteils kleiner als ein innerer Durchmesser des Wellenlumens (11) ist.

3. Katheter nach Anspruch 2, bei dem die distale Spitze (16) aus dem ersten Material gebildet ist.

4. Katheter nach Anspruch 1, bei dem die distale Spitze (16) aus einem dritten Material gebildet ist, wobei das erste Material steifer als das dritte Material ist.

5. Katheter nach Anspruch 1, bei dem das Wellenlumen (11) unmittelbar proximal von dem Anlageteil (70) einen konischen Übergang (72) enthält, wobei der innere Durchmesser eines Teils des Wellenlumens (11) von der proximalen Öffnung zu einem proximalen Ende des konischen Übergangs (72) im Wesentlichen konstant bleibt und durch den konischen Übergang (72) allmählich abnimmt.

6. Katheter nach Anspruch 5, bei dem ein innerer Durchmesser des Lumens (11') des Anlageteils über seine Länge im Wesentlichen konstant ist.

## Revendications

1. Cathéter (10) incluant : un arbre (12) formé en un premier matériau, l'arbre (12) incluant une lumière d'arbre (11) s'étendant sur toute sa longueur depuis une ouverture proximale jusqu'à une extrémité distale de l'arbre (12) ;
un élément de butée (60, 70) couplé à l'extrémité distale de l'arbre (12), l'élément de butée (60, 70) étant formé en un second matériau plus raide que le premier matériau et formant une surface de butée recouvrant au moins partiellement une extrémité distale de la lumière d'arbre (11) ; et
une pointe distale (16) couplée à l'élément de butée (60, 70), la pointe distale (16) incluant une lumière de pointe (11') s'étendant sur toute sa longueur jusqu'à une ouverture distale,
**caractérisé en ce que** :
l'élément de butée (60, 70) s'étend au travers de la pointe distale (16) jusqu'à une extrémité au niveau de l'ouverture distale de la pointe distale (16).

2. Cathéter selon la revendication 1, dans lequel un diamètre interne de la lumière d'élément de butée (11') est inférieur à un diamètre interne de la lumière d'arbre (11).

3. Cathéter selon la revendication 2, dans lequel la pointe distale (16) est formée en le premier matériau.

4. Cathéter selon la revendication 1, dans lequel la pointe distale (16) est formée en un troisième matériau, le premier matériau étant plus raide que le troisième matériau.

5. Cathéter selon la revendication 1, dans lequel la lumière d'arbre (11) inclut une transition à flancs inclinés (72) immédiatement proximale par rapport à l'élément de butée (70), le diamètre interne d'une partie de la lumière d'arbre (11) restant sensiblement constant depuis l'ouverture proximale jusqu'à une extrémité proximale de la transition à flancs inclinés (72) et augmentant progressivement au fil de la transition à flancs inclinés (72).

6. Cathéter selon la revendication 5, dans lequel un diamètre interne de la lumière d'élément de butée (11') est sensiblement constant sur toute sa longueur.
